Europäisches Patentamt

European Patent Office

Office européen des brevets

(19)

(11) Publication number: **0 439 643 A1**

## EUROPEAN PATENT APPLICATION

(21) Application number: 90101727.7

(22) Date of filing: 29.01.90

(51) Int. Cl.5: **A61K 33/00**

(43) Date of publication of application:
07.08.91 Bulletin 91/32

(84) Designated Contracting States:
AT BE CH DE DK ES FR GB GR IT LI LU NL SE

(71) Applicant: "HARRIER" GMBH GESELLSCHAFT
FÜR DEN VERTRIEB MEDIZINISCHER UND
TECHNISCHER GERÄTE
Prälat-Zistl-Strasse 6
W-8000 München 2(DE)

(72) Inventor: Kolta, Peter
Kand k. 11
7623 Pécs(HU)
Inventor: Lantos, Michael
Csopaki U. 5/A
1022 Budapest(HU)
Inventor: Ulrich, Armando
Bergstrasse 59
CH-5644 Auw(CH)

(74) Representative: Patentanwälte Viering &
Jentschura
Steinsdorfstrasse 6
W-8000 München 22(DE)

(54) Physiologic solution with increased oxygen content and a method for preparing the same.

(57) Physiologic solution with increased oxygen content and a method for preparing the same. The physiologic solution for treating living tissues, comprising potassium chloride, sodium chloride and if required additional components, which together provide an ionic composition substantially corresponding to the ionic composition of said tissues is characterized by comprising oxygen in an amount exceeding saturation level. The method for preparing a physiologic solution having a predetermined composition including potassium chloride and sodium chloride, is characterized by the steps of providing distilled water, dissolving at least partially the potassium chloride component in said distilled water to provide a first liquid, enriching the first liquid in oxygen by molecularly bonding oxygen to water molecules present in said first liquid to obtain an oxygen concentration exceeding saturation level, providing a stock solution comprising the sodium chloride and optional other salty components of the physiologic solution to be prepared, adding said stock solution to said enriched liquid, and adding a buffer solution for keeping the pH value of the physiologic solution within a predetermined tolerance range.

## PHYSIOLOGIC SOLUTION WITH INCREASED OXYGEN CONTENT AND A METHOD FOR PREPARING THE SAME

The invention relates to a novel physiologic solution and to a method for preparing this solution.

The term "physiologic solution" is well known in the medical and veterinarian field of art and it designates various kinds of solutions which comprise different salts and ions which imitate the natural concentration of such materials in the extracellular liquids of living organisms and of that reason these solutions can be used in the form of infusion without disturbing the ionic balance of such organisms. The composition of such solutions differ from each other depending on various factors. One of these is the type or order· of the organization to which the solution can be applied. The ion concentration is largely different in case of amphibians, reptiles, birds, mammals and human subjects and the physiologic solutions follows these differences. Slight differences arise from the intended use of the solutions. Certain kinds of physiologic solutions bear the name of the inventors e.g. Ringer, Locke or Tyrode solutions. These solutions are prepared mainly in such a way that the salts are dissolved in double distilled water and a buffer solution is added to the liquid in such an amount which can ensure that the pH of the solution remains within a predetermined range. The manufacturing process is generally followed be an appropriate sterilization step which can be the exposure of the liquid to increased temperatures or the use of a microfilter.

There is a common characteristic of each type of physiologic solution i.e. they do not participate directly in the cellular metabolism, more particularly they neither pass oxygen to the tissues nor take oxygen away therefrom.

The object of the invention is to provide a physiologic solution which, in addition to various conventional components comprises oxygen in an amount exceeding saturation level (which is about 40-50 mg/l) in a form readily utilizable by the cells getting into contact with the solution. A related object of the invention is to provide a method for preparing such solution.

In the international publication WO 89/03724 a water comprising excess quantity of oxygen has been described. For the sake of simplicity such a water will be referred to as 'oxy-water'.

One might think that the object of the invention can be readily achieved by substituting oxy-water for the distilled water in the conventional methods of making physiologic solutions. This is, however, not the case and a number of difficulties should be overcome before 'oxy-water' could be utilized for such purposes.

Before disclosing the inventive idea a few number of such difficulties will be explained.

It is known in the field of physico-chemistry that the presence of certain salts, mainly of sodium, assists in removing gases present in the water in dissolved form. This phenomenon can be attributed to the structure maker property of sodium ions. Physiologic solutions comprise such structure maker ions in large quantities, therefore their addition to the basic liquid would decrease the amount of excess oxygen if oxy-water was used as basic liquid.

Owing to the low level bonding forces between oxygen and water molecules in oxy-water as explained in the above referred international publication the sterilization of oxy-water cannot be carried out at excess temperatures, since at such temperatures substantial portion of the oxygen would escape from the liquid.

If microfiltration technique was chosen for sterilizing the liquid, this would impose further difficulties, since the increased air pressure used at such technique for driving the liquid through the pores of the filter material has the tendency of decreasing oxygen concentration as well. It can be appreciated, however, that a kind of sterilization is a prerequisite of using a physiologic solution under in vivo circumstances, therefore a way for sterilizing the physiologic solution without jeopardizing the excess amount of oxygen should also be provided.

According to a first aspect of the invention it has been found that these difficulties can at least partially be eliminated if the physiologic solution is made from distilled water as a starting liquid in such a way that the potassium chloride component of the physiologic solution to be made is added first and with the presence of this component oxygen is bound to the liquid especially as described in the international publicaion WO 89/03724, the disclosure thereof being hereby included into the present specification by reference thereto. By said oxygen bonding step a liquid comprising oxygen in a quantity exceeding saturation level and preferably exceeding 100 g/l is formed. The potassium chloride is a "structure breaker" material, and its presence decreases internal structure of water and in this way assists in the process of forming hydrogen bridges which capture oxygen in the specific way as described in the above referred publication. In principle, other structure breaker materials could also be used instead of potassium chloride, however, this latter material should always be present in physiologic solutions, therefore its addition cannot change the final composition of such physiologic solution. This explains why the choice of potassium chloride is preferred.

The remaining salts of the physiologic solution to be prepared should be dissolved first in distilled water in a separate tank. This solution should be as concentrated in salts as possible, therefore it forms a stock solution. The reason of using highly concentrated stock solution lies in that the salts required to form the physiologic solution will be added by pouring this stock solution to the liquid comprising excess oxygen made in the first step. If the stock solution were less concentrated, than the first solution would become more diluted. It can be appreciated that dilution decreases oxygen concentration which is against our main objective i.e. to prepare a physiologic solution which has maximum amount of oxygen.

In the concentrated stock solution the structure maker ions like sodium ions will be surrounded by polarized water molecules and the presence of such molecules neutralizes the structure maker effect of such ions. If the structure maker salts are poured in the physiologic solution in such a "neutralized" state, then they cannot effectively remove oxygen present in the liquid.

In a third step the conventional buffer solution should be added which has the task of preserving pH of the final solution.

In a preferable embodiment gases which might be present in the distilled water used in the first step as the main liquid should be removed before the addition of potassium chloride. The gas removal can be realized by boiling the water in a closed environment and letting gases be removed together with vapor through a water trap. The distilled water from which gases have been removed cannot be exposed to the free atmosphere any more.

The physiologic solution enriched in oxygen will be referred to hereinafter as KS solution for being distinguished from normal physiologic solutions, and it must be sterilized before use.

According to a further aspect of the invention this sterilization should be done in a conventional microfilter apparatus in such a way that the liquid is driven through the filter plate under the pressure of compressed oxygen gas instead of compressed air.

It is preferred if the so-obtained KS solution is stored in sealed flasks and if pure oxygen fills the space above the liquid level.

The KS solution made according to the invention differs from conventional physiologic solutions in that it comprises oxygen in an amount exceeding normal saturation level and preferably exceeding 100 mg/l. It should be noted, that in human body venal blood has an oxygen content of about 100 mg/l. Tissues can utilize oxygen connected to hemoglobin only if the partial pressure of oxygen in blood is higher than that in the interstitial field. In well functioning organizations the respiratory system supplies sufficient amount of oxygen which get bound to the hemoglobin, whereby the blood will have an oxygen concentration varying between about 100 and 200 mg/l.

It has been tested and verified that living tissues can utilize the oxygen content of the KS solution if its oxygen concentration exceeds the one in the interstitial field. This phenomenon can form the basis of different and novel fields of applications.

The invention will now be described in connection with examples.

Example 1

One liter double distilled water was boiled in a closed space and vapor and steam was lead out through a tube immersed in water contained in a separate water tank. The free surface of the water in the tank was covered by a thin paraffine film to prevent the dissolution of air therein. Any gas contained in the distilled water escaped therefrom by bubbling out through the water seal in the second tank. The double distilled water was cooled to room temperature and 0.2 g potassium chloride (KCl) was dissolved therein.

The water was then filled in the water tank of an oxygenizing apparatus as shown in Fig. 10 of the above referred international publication WO 89/0374, it was cooled down to 16° C and the apparatus was set into operation as described in that publication. During this process oxygen was taken up by the water. This process went on until the oxygen concentration reched a value of about 140 mg/l. This 1 liter liquid formed the first (basic) component of the KS solution.

In a separate bottle containing 50 ml of double distilled water a concentrated salt solution was made by adding 8 g sodium chloride (NaCl), 0.2 g calcium chloride ($CaCl_2$) in powdered form, 0.1 g magnesium chloride ($MgCl_2$). After an intensive mixing step the salts were dissolved and the so obtained liquid formed the second component i.e. stock solution.

A third (buffer) solution was made by adding 1 g of sodium hydro chloride ($NaHCO_3$) to 50 ml distilled water. This liquid formed the third (buffer) component of the KS solution.

In the next step 900 ml was taken from the first component to which the second and the third components were added, whereby 1000 ml KS solution comprising about 125 mg/l oxygen was obtained. This was an isoionic solution usable for human applicaions.

It should be noted that for tests made with frogs the amount of potassium chloride was substantially less, it was 0.02 g/l.

Example 2

A KS solution was made for experiments with rats. The 900 ml first component and the 50 ml third (buffer) component was made just as in Example 1. The second component i.e. the stock solution was 50 ml distilled water, in which the following materials were dissolved:

4.8 g Na lactate

6 g NaCl

0.3 g $CaCl_2$

1 g glucose

The 50 ml stock solution and the 50 ml buffer solution were added to the 900 ml first component, whereby a KS solution for rats was obtained.

Of course, several other KS solutions can be made than disclosed in these two examples. In the medical literature numerous isotonic and isoionic (physiologic) solutions are described which differ in certain components depending on the intended use and on the type of the living organism in which the use is envisaged. All of these solutions can be enriched in oxygen in the way disclosed hereinabove, since the method is not influenced by the materials in the stock and buffer solutions.

Example 3

The KS solution obtained either from examples 1 or 2 should be sterilized or more exactly the number of bacteria should be decreased below a predetermined threshold level.

This has been done in a Sartorius type microfilter in such a way that the KS solution was introduced in the primary side of the apparatus through a magnet valve. After closing the magnet valve the KS solution was exposed through a further magnet valve to the effect of pure oxygen under the pressure of about 2 bars. This pressure was sufficiently high to drive the KS liquid through the filter plate of the apparatus which withheld bacterii being accidentally present in the liquid. The presence of oxygen as driving gas instead of compressed air prevented the saturation of the KS solution with air which, owing to the 80% nitrogen content of air, would decrease the amount of dissolved oxygen in the KS solution.

**Claims**

1. Physiologic solution for treating living tissues, comprising potassium chloride, sodium chloride and if required additional components, which together provide an ionic composition substantially corresponding to the ionic composition of said tissues, characterized by comprising oxygen in an amount exceeding saturation level.

2. The physiologic solution as claimed in claim 1, characterized in that the oxygen concentration exceeds 100 mg/l.

3. A method for preparing a physiologic solution having a predetermined composition including potassium chloride and sodium chloride, characterized by the steps of:
   - providing distilled water,
   - dissolving at least partially the potassium chloride component in said distilled water to provide a first liquid,
   - enriching the first liquid in oxygen by molecularly bonding oxygen to water molecules present in said first liquid to obtain an oxygen concentration exceeding saturation level,
   - providing a stock solution comprising the sodium chloride and optional other salty components of the physiologic solution to be prepared,
   - adding said stock solution to said enriched liquid, and
   - adding a buffer solution for keeping the pH value of the physiologic solution within a predetermined tolerance range.

4. The method as claimed in claim 4, wherein gases are removed from said distilled water before the addition of said potassium chloride.

5. The method as claimed in claims 3 or 4, wherein said physiologic solution is sterilized by driving said solution through a microfilter by means of a compressed gas.

6. The method as claimed in claim 5, wherein said compressed gas is oxygen.

7. The method as claimed in claims 5 or 6, wherein said physiologic solution is stored in sealed flasks and oxygen fills the space above the liquid level.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | AM. J. PHYSIOLO, vol. 245, 1983, pages H653-H661, American Physiological Society; C.M. HEESCH et al.: "Effects of calcium channel blockers on isolated carotid baroreceptors and baroreflex" * Page H654, "Solutions and drugs" * | 1-7 | A 61 K 33/00 |
| A | CHEMICAL ABSTRACTS, vol. 83, no. 24, 15th December 1975, page 290, abstract no. 197772w, Columbus, Ohio, US; G. MARX: "Oxygenation of physiological solutions in a pulsed capillary membrane oxygenator", & JAHRESTAG. DTSCH. GES. BIOMED. TECH. 1973, 28 * Abstract * | 1-7 | |
| D,A | WO-A-8 903 724 (HARRIER GmbH) * Whole document * | 1-7 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 16-08-1990 | BERTOCCHI C. |

EPO FORM 1503 03.82 (P0401)